# EUROPÄISCHE PATENTSCHRIFT

(11) **EP 0 717 028 B1**
(45) Veröffentlichungstag und Bekanntmachung des Hinweises auf die Patenterteilung: **31.03.1999**
(21) Anmeldenummer: 95119855.5
(22) Anmeldetag: 15.12.1995
(51) Int. Cl.: C07C 53/02, C07C 51/48, C07C 51/09

(54) **Verfahren und Vorrichtung zur Gewinnung von Ameisensäure**
Method and apparatus for obtaining formic acid
Procédé et appareil permettant d'obtenir de l'acide formique

(30) Priorität: 16.12.1994 DE 4444979
(43) Veröffentlichungstag der Anmeldung: 19.06.1996
(73) Patentinhaber: BASF AKTIENGESELLSCHAFT, 67056 Ludwigshafen (DE)
(72) Erfinder: Hammer, Hans, D-68219 Mannheim (DE); Herr, Manfred, D-67157 Wachenheim (DE); Hupfer, Leopold, Dr., D-67159 Friedelsheim (DE); Bessling, Bernd, D-67269 Grünstadt (DE); Pan, Dongfeng, Dr., D-68167 Mannheim (DE); Zeck, Sebastian, Dr., D-67251 Freinsheim (DE); Auer, Heinz, D-68809 Neulussheim (DE); Schoenmakers, Hartmut, Dr., D-69121 Heidelberg-Handschuhsheim (DE)
(74) Vertreter: Isenbruck, Günter, Dr.

(56) Entgegenhaltungen:
- EP-A- 0 017 866
- EP-A- 0 127 836
- DE-A- 2 545 730

## Beschreibung

Die vorliegende Erfindung betrifft ein Verfahren und eine Vorrichtung zur Gewinnung von Ameisensäure, insbesondere durch die Hydrolyse von Methylformiat.

Es ist bekannt, Ameisensäure durch die Hydrolyse von Methylformiat herzustellen, das man seinerseits aus der Reaktion von Kohlenmonoxid mit Methanol gewinnt.

Nach der Lehre des europäischen Patents 0 017 866 gewinnt man Ameisensäure, indem man Methylformiat hydrolysiert, vom erhaltenen Hydrolysegemisch das Methanol sowie das überschüssige Methylformiat abdestilliert, dem Sumpfprodukt Wasser in einer Flüssig-flüssig-Extraktion entzieht, das erhaltene Kopfprodukt von Ameisensäure, Extraktionsmittel und Restwasser einer weiteren Destillierkolonne zuführt und das weitgehend wasserfreie Sumpfprodukt dieser zweiten Destillation zur Abtrennung des Extraktionsmittels von der Ameisensäure in einer dritten Destillierkolonne behandelt. Auf diese Weise wird großtechnisch Ameisensäure mit einem Ameisensäuregehalt von etwa 95 Gew.-% erzeugt. Da Nachfrage vor allem nach etwa 85%iger und etwa 99%iger Ameisensäure besteht, muß die erhaltene Ameisensäure einerseits nochmals hochdestilliert, andererseits mit Wasser abgemischt werden. Dabei stößt man bei der Aufdestillierung von bereits hochkonzentrierter Ameisensäure auf erhebliche technische Schwierigkeiten, die vor allem von der hohen Korrosivität hochkonzentrierter Ameisensäure herrühren. Da die Nachfrage nach 85%iger Arneisensäure erheblich größer ist als der Bedarf an 99%iger Ameisensäure, kann die Lösung auch nicht einfach in einer weiteren Aufpegelung der gesamten Ameisensäure in der dritten Destillierkolonne liegen. Eine höhere Aufkonzentrierung der Ameisensäure setzt außerdem eine höhere Ameisensäurekonzentration im Sumpf der zweiten Destillierkolonne voraus. Eine höhere Konzentration der Ameisensäure im Sumpf der zweiten Destillierkolonne kann aber nur mit höheren Temperaturen erreicht werden. Dies führt wiederum zu stark erhöhter Zersetzung der Ameisensäure und damit zu erheblichen Produktverlusten.

Aufgabe der Erfindung ist deshalb die Bereitstellung eines Verfahrens zur einfachen Herstellung von unterschiedlich konzentrierten Ameisensäuren in einem Verfahrenszug, sowie eine Vorrichtung zur Durchführung dieses Verfahrens. Insbesondere soll ein Verfahren bereitgestellt werden, das bei gleicher Gesamtenergiebilanz die Herstellung von 85%iger und 99%iger Ameisensäure in einem Arbeitsgang erlaubt und die temperaturbedingte Zersetzung von Ameisensäure im Sumpf einer Extraktivdestillierkolonne vermeidet.

Diese Aufgabe wird durch das in den Patentansprüchen beschriebene Verfahren gelöst. Zur Gewinnung von wasserfreier oder weitgehend wasserfreier Ameisensäure unterwirft man dabei Methylformiat der Hydrolyse. Vom erhaltenen Hydrolysegemisch destilliert man das Methanol sowie das überschüssige Methylformiat ab. Das aus Ameisensäure und Wasser bestehende Sumpfprodukt dieser Destillation extrahiert man in einer Flüssig-flüssig-Extraktion mit einem hauptsächlich die Ameisensäure aufnehmenden Extraktionsmittel. Die dabei erhaltene, aus Ameisensäure, dem Extraktionsmittel und einem Teil des Wassers bestehende Extraktphase unterwirft man einer Destillation. Das bei dieser Destillation erhältliche, aus der Gesamtmenge oder einer Teilmenge des in die Destillation eingeführten Wassers und einem Teil der Ameisensäure bestehende Kopfprodukt führt man dampfförmig in den unteren Teil der ersten Destillierkolonne zur Abdestillation von Methanol und Methylformiat aus dem Hydrolysegemisch zurück. Das aus dem Extraktionsmittel, gegebenenfalls einer Teilmenge des Wassers und dem Großteil der Ameisensäure bestehende Sumpfprodukt der zweiten Destillationsstufe trennt man anschließend destillativ in wasserfreie bzw. weitgehend wasserfreie Ameisensäure sowie in das Extraktionsmittel auf. Das Verfahren ist dadurch gekennzeichnet, daß man in der letzten Verfahrensstufe über mindestens einen Seitenabzug einen oder mehrere flüssige Ströme mit einem Ameisensäureanteil von 80 bis 95 Gew.-% und über einen Kopfabzug einen Strom mit einem Ameisensäureanteil von mindestens 95 Gew.-%, insbesondere 99 Gew.-%, entnimmt.

Vorzugsweise führt man dabei in der letzten Verfahrensstufe mindestens einen Ameisensäurestrom aus dem Seitenabzug oder einen Teil davon in die Destillierkolonne zur Abdestillation des Wassers zurück.

Bevorzugt wird dabei das die letzte Destillationsstufe verlassende Extraktionsmittel in das Verfahren zurückgeführt. Vorzugsweise werden der erste und der zweite Destillationsschritt in einer einzigen Kolonne vorgenommen, die die Funktionen der Destillierkolonnen beider Schritte erfüllt.

Weiter bevorzugt ist ein Verfahren, bei dem man das für die Hydrolyse benötigte Wasser dampfförmig in den unteren Teil der ersten Destillierkolonne einbringt. Bei Ausführung des ersten und des zweiten Destillationsschrittes in einer einzigen Kolonne wird das für die Hydrolyse benötigte Wasser dampfförmig in den mittleren Teil der Kolonne eingebracht.

Bevorzugt ist weiterhin der Einsatz von Methylformiat und Wasser bei der Hydrolyse im Molverhältnis 1:2 bis 1:10. Vorzugsweise verwendet man als Extraktionsmittel ein Carbonsäureamid der allgemeinen Formel (**I**) 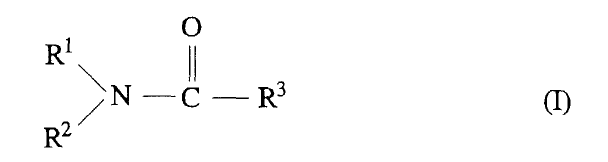 in der die Reihe R¹ und R² Alkyl-, Cycloalkyl-, Aryl- oder Aralkylgruppen oder gemeinsam eine 1,4- oder 1,5-Alkylengruppe mit jeweils 1 bis 8 C-Atomen mit der Maßgabe bedeuten, daß die Summe der C-Atome von R¹ und R² 7 bis 14 beträgt und daß nur einer der Reste eine Arylgruppe ist. R³ steht vorzugsweise für Wasserstoff oder eine C₁-C₄-Alkylgruppe. Vorzugsweise wird dabei die Hydrolyse in Gegenwart des Extraktionsmittels vorgenommen.

Die erfindungsgemäße Aufgabe wird auch durch die in den Patentansprüchen beschriebene Vorrichtung gelöst. Diese Vorrichtung zur Durchführung eines erfindungsgemäßen Verfahrens weist dabei folgende Bestandteile auf: einen Reaktor H zur Hydrolyse von Methylformiat, eine Destillierkolonne D1 zum Abdestillieren von Methanol aus dem Hydrolysegemisch, eine Extraktionskolonne E zum Extrahieren von Wasser aus dem Sumpfprodukt der Destillierkolonne D1, eine Destillierkolonne D2 zum weiteren Entwässern der extrahierten Mischung aus Ameisensäure und Extraktionsmittel und eine Destillierkolonne D3 zum Abdestillieren des Extraktionsmittels aus dem Sumpfprodukt der Destillierkolonne D2. Die Vorrichtung ist durch mindestens einen Seitenabzug und einen Kopfabzug an der Destillierkolonne D3 zum Entnehmen von Ameisensäuren unterschiedlicher Konzentration gekennzeichnet.

Bevorzugt weist die Vorrichtung mindestens eine Leitungseinrichtung zwischen mindestens einem Seitenabzug der Destillierkolonne D3 und der Destillierkolonne D2 zur Rückführung von Ameisensäure auf.

Vorzugsweise sind die Destillierkolonnen D1 und D2 als eine Destillierkolonne ausgebildet.

Die Erfindung soll nun anhand folgender Figuren erläutert werden. Dabei zeigen:
- Fig. 1: eine schematische Darstellung eines erfindungsgemäßen Verfahrens bzw. einer erfindungsgemäßen Vorrichtung,
- Fig. 2: eine schematische Darstellung eines weiteren erfindungsgemäßen Verfahrens bzw. einer weiteren erfindungsgemäßen Vorrichtung,
- Fig. 3: das Ablaufdiagramm der Herstellung von unterschiedlich aufkonzentrierten Ameisensäuren nach dem Stand der Technik,
- Fig. 4: das Ablaufdiagramm der Herstellung von unterschiedlich aufkonzentrierten Ameisensäuren nach der vorliegenden Erfindung.

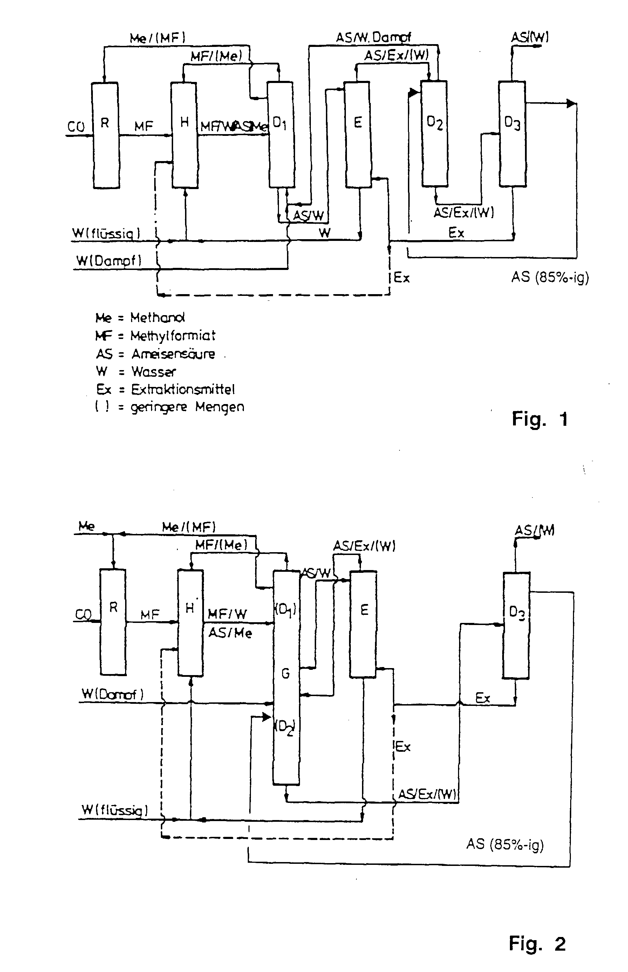

Fig. 1 zeigt den schematischen Aufbau eines erfindungsgemäßen Verfahrens bzw. einer erfindungsgemäßen Vorrichtung zur Herstellung von wasserfreier oder weitgehend wasserfreier Ameisensäure. In dem Reaktor R wird Methylformiat aus Methanol und Kohlenmonoxid hergestellt. Das gewonnene Methylformiat wird gemeinsam mit flüssigem Wasser dem Hydrolysereaktor H zugeführt, wobei die Hydrolyse bei 80 bis 150°C vorgenommen wird. Das Hydrolysegemisch, bestehend aus Methylformiat, Wasser, Ameisensäure und Methanol, wird anschließend der Destillierkolonne D1 zugeleitet, in welcher Methylformiat und Methanol von der wäßrigen Ameisensäure abdestilliert werden. Die Destillation erfolgt bei Drücken von 0,5 bis 2 bar, vorzugsweise bei Normaldruck; die Temperatur im Kolonnensumpf beträgt dabei etwa 110°C, am Kolonnenkopf beträgt sie 30 bis 40°C. Zweckmäßigerweise wird das Hydrolysegemisch im Temperaturbereich von 80 bis 150°C zugegeben; das abdestillierte Methanol entnimmt man vorzugsweise flüssig im Bereich von 55 bis 65°C. Dabei zieht man zweckmäßigerweise das Methylformiat, das nicht vollständig methanolfrei zu sein braucht, über Kopf ab und leitet es in den Hydrolysereaktor H zurück. Ebenso entnimmt man das Methanol, welches noch etwas Methylformiat enthalten kann, als Seitenstrom und führt dieses in den Synthesereaktor R zurück. Das durch Destillation erhaltene Methanol bzw. Methylformiat sollte jeweils eine Reinheit von 90 Gew.-% aufweisen, um für die Wiederverwendung zur Herstellung von Methylformiat bzw. zur Hydrolyse geeignet zu sein.

Das in dieser ersten Destillation erhaltene Sumpfprodukt aus Ameisensäure und Wasser wird der Flüssig-flüssig-Extraktionskolonne E zugeführt, wo es von einem Extraktionsmittel im Gegenstrom durchsetzt wird. Das abgetrennte Wasser sammelt sich im Sumpf der Extraktionskolonne und kann zur Wiederverwendung in den Hydrolysereaktor H zurückgeführt werden. Das Ameisensäure/Extraktionsmittel-Gemisch, das auch noch Wasser enthält, wird über Kopf abgezogen und der zweiten Destillierkolonne D2 zugeführt.

Geeignete Extraktionsmittel sind alle Flüssigkeiten, in denen sich die Ameisensäure löst und die mit Wasser nicht oder wenig mischbar sind. Besonders geeignete Extraktionsmittel sind vor allem N-Di-n-butylformamid, N-Di-n-butylacetamid, N-Methyl-N-2-heptylformamid, N-n-Butyl-N-2-ethylhexylformamid, N-n-Butyl-N-cyclohexylformamid, N-Ethylformanilid, sowie Gemische dieser Verbindungen. Weitere geeignete Extraktionsmittel sind unter anderem Diisopropylether, Methylisobutylketon, Ethylacetat, Tributylphosphat und Butandiolformiat.

Die der Destillierkolonne D2 zugeleitete Extraktphase wird dort in eine Flüssigphase, die aus Ameisensäure, Extraktionsmittel und, sofern man eine wasserhaltige Ameisensäure gewinnen will, Wasser besteht, sowie in eine Dampfphase aus Wasser und geringen Mengen Ameisensäure zerlegt. Diese Dampfphase wird aus der Destillierkolonne D2 über Kopf abgezogen und in den Sumpf der Destillierkolonne D1 zurückgeführt. Die im Sumpf gesammelte Flüssigphase wird schließlich der Destillierkolonne D3 zugeleitet, wo eine destillative Auftrennung in Extraktionsmittel und Ameisensäure stattfindet. Erfindungsgemäß ist sowohl ein Seitenabzug wie auch ein Kopfabzug vorgesehen, so daß Ameisensäure verschiedener Gewichtskonzentration in diesem Verfahrensschritt gewonnen werden kann. Die über den Seitenabzug entnommene, mindestens 85%ige Ameisensäure kann entweder als vermarktungsfertiges Produkt abgeführt oder aber der Destillierkolonne D2 wieder zugeführt werden. Diese Ameisensäurerückführung führt zu einer höheren Ameisensäurekonzentration im Sumpf der Destillierkolonne D2. Bei unveränderter Sumpftemperatur in der Destillierkolonne D2 ist somit eine höhere Aufkonzentrierung der Ameisensäure in der Destillierkolonne D3 möglich. Durch eine Begrenzung der Sumpftemperatur in D2 wird dort eine Zersetzung der Ameisensäure vermieden, die zu erheblichen Produktverlusten führt.

Das in D3 abgetrennte Extraktionsmittel wird zu der Extraktionskolonne zurückgeleitet. Bei Verwendung eines Extraktionsmittels der allgemeinen Formel (**I**) 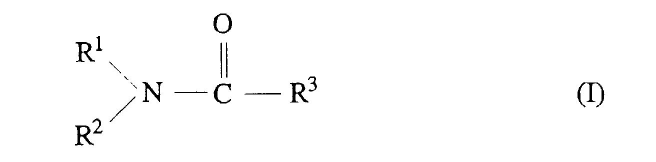 in der die Reste R¹ und R² Alkyl-, Cycloalkyl-, Aryl- oder Aralkylgruppen sind oder gemeinsam eine 1,4- oder 1,5-Alkylengruppe mit jeweils 1 bis 8 C-Atomen mit der Maßgabe bedeuten, daß die Summe der C-Atome von R¹ und R² 7 bis 14 beträgt und daß nur einer der Reste eine Arylgruppe ist, und in der R³ für - vorzugsweise Wasserstoff - oder eine C₁-C₄-Alkylgruppe steht, ist es vorteilhaft, die Hydrolyse in Gegenwart des Extraktionsmittels ablaufen zu lassen. Dadurch verschiebt sich das Gleichgewicht im Hydrolyseschritt zugunsten der Ameisensäure. Extraktionsmittel wird in diesem Fall aus dem Sumpf der Destillierkolonne D3 auch zum Hydrolysereaktor H zurückgeleitet. Vorzugsweise setzt man dabei 0,5 bis 2 Mol Extraktionsmittel pro Mol Methylformiat ein. Die bevorzugte Wassermenge beträgt in diesem Falle 0,5 bis 2 Mol pro Mol Methylformiat.

Fig. 2 zeigt das Prinzip eines weiteren erfindungsgemäßen Verfahrens bzw. einer weiteren erfindungsgemäßen Vorrichtung, wobei die Destillierkolonnen D1 und D2 zu einer einzigen Destillierkolonne D vereinigt sind, die die Funktionen der beiden Destillierkolonnen D1 und D2 übernimmt.

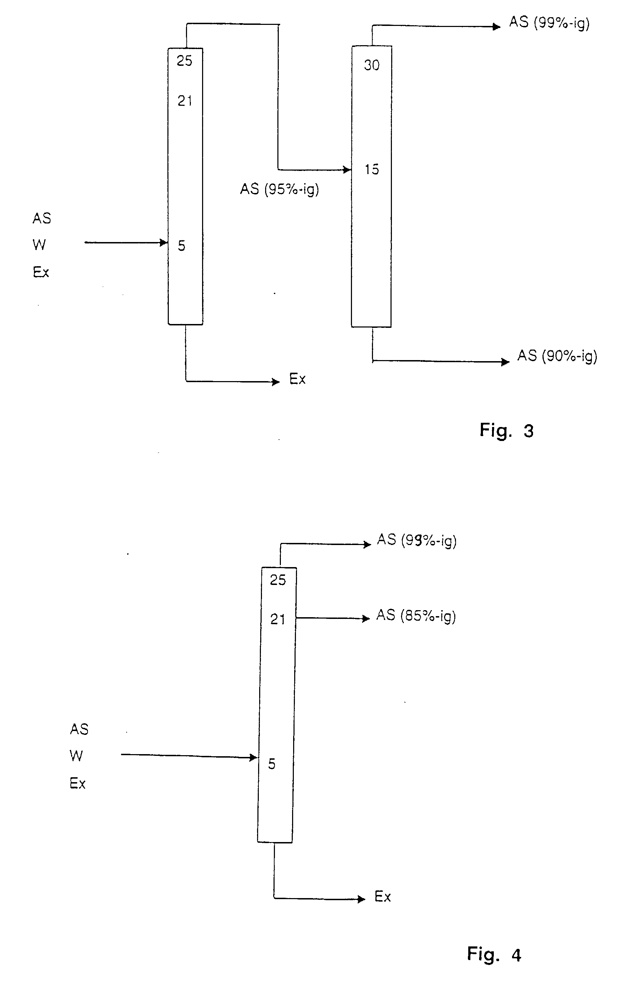

Fig. 3 zeigt den schematischen Ablauf der Ameisensäureherstellung nach dem Stand der Technik. Um etwa 90%ige und etwa 99%ige Ameisensäure zu erhalten, muß die typischerweise 95%ige Ameisensäure nach Verlassen der Destillierkolonne D3 nochmals einerseits aufkonzentriert und andererseits abgemischt werden.

Fig. 4 zeigt die Gewinnung von Ameisensäuren unterschiedlicher Konzentration nach dem erfindungsgemäßen Verfahren in einem Schritt. Dabei wird aus der Destillierkolonne D3 über einen Seitenabzug die niedriger konzentrierte Ameisensäure entnommen, während über den Kopfabzug die gewünschte hochkonzentrierte Ameisensäure abgeleitet werden kann.

## Patentansprüche

1. Verfahren zur Gewinnung von wasserfreier oder weitgehend wasserfreier Ameisensäure durch Hydrolyse von Methylformiat,
wobei man
a. Methylformiat der Hydrolyse unterwirft,
b. vom erhaltenen Hydrolysegemisch das Methanol sowie das überschüssige Methylformiat abdestilliert,
c. das aus Ameisensäure und Wasser bestehende Sumpfprodukt der Destillation (b) in einer Flüssig-flüssig-Extraktion mit einem hauptsächlich die Ameisensäure aufnehmenden Extraktionsmittel extrahiert,
d. die hierbei erhaltene, aus Ameisensäure, dem Extraktionsmittel und einem Teil des Wassers bestehende Extraktphase einer Destillation unterwirft,
e. das bei dieser Destillation erhältliche, aus der Gesamtmenge oder einer Teilmenge des in die Destillation eingeführten Wassers und einem Teil der Ameisensäure bestehende Kopfprodukt dampfförmig in den unteren Teil der Destillationskolonne der Stufe (b) zurückführt,
f. das aus dem Extraktionsmittel, gegebenenfalls einer Teilmenge des Wassers und dem Großteil der Ameisensäure bestehende Sumpfprodukt der Destillationsstufe (d) destillativ in unterschiedlich hoch konzentrierte flüssige Ströme von wasserfreier bzw. weitgehend wasserfreier Ameisensäure, sowie in das Extraktionsmittel auftrennt,
**dadurch gekennzeichnet, daß**
man in Stufe (f) über mindestens einen Seitenabzug einen oder mehrere flüssige Ströme mit einem Ameisensäureanteil von 80 bis 95 Gew.-%, und über einen Kopfabzug einen flüssigen Strom mit einem Ameisensäureanteil von mindestens 95 Gew.-% entnimmt.

2. Verfahren nach Anspruch 1, dadurch gekennzeichnet, daß man das die Stufe (f) verlassende Extraktionsmittel in den Verfahrensgang zurückführt.

3. Verfahren nach Anspruch 1, dadurch gekennzeichnet, daß man die Destillationsschritte (b) und (d) in einer einzigen Kolonne vornimmt, welche die Funktionen der Kolonnen dieser Schritte erfüllen.

4. Verfahren nach Anspruch 1, dadurch gekennzeichnet, daß man das für die Hydrolyse benötigte Wasser dampfförmig in den unteren Teil der Kolonne des Schrittes (b) einbringt.

5. Verfahren nach Anspruch 2, dadurch gekennzeichnet, daß man das für die Hydrolyse benötigte Wasser dampfförmig in den mittleren Teil der Kolonne einbringt.

6. Verfahren nach den Ansprüchen 1 bis 4, dadurch gekennzeichnet, daß man bei der Hydrolyse (a) Methylformiat und Wasser im Molverhältnis 1:2 bis 1:10 einsetzt.

7. Verfahren nach den Ansprüchen 1 bis 5, dadurch gekennzeichnet, daß man als Extraktionsmittel ein Carbonsäureamid der allgemeinen Formel **I** 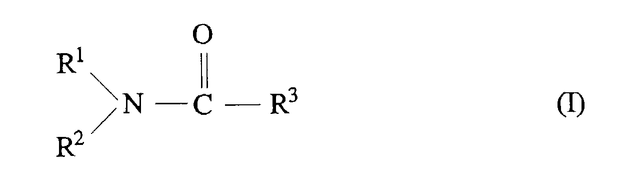 verwendet, in der die Reihe R¹ und R² Alkyl-, Cycloalkyl-, Aryl- oder Aralkylgruppen oder gemeinsam eine 1,4- oder 1,5-Alkylengruppe mit jeweils 1 bis 8 C-Atomen mit der Maßgabe bedeuten, daß die Summe der C-Atome von R¹ und R² 7 bis 14 beträgt und daß nur einer der Reste eine Arylgruppe ist, und in der R³ für - vorzugsweise wasserstoff - oder eine C₁-C₄-Alkylgruppe steht.

8. Verfahren nach den Ansprüchen 1 bis 6, dadurch gekennzeichnet, daß man im Falle der Verwendung eines Extraktionsmittels (I) die Hydrolyse (a) in Gegenwart des Extraktionsmittels vornimmt.

9. Vorrichtung zur Durchführung eines Verfahrens nach einem der Verfahrensansprüche, wobei die Vorrichtung aufweist:
einen Reaktor (H) zur Hydrolyse von Methylformiat,
eine Destillierkolonne (D1) zum Abdestillieren von Methanol aus dem Hydrolysegemisch,
eine Extraktionskolonne (E) zum Extrahieren von Wasser aus dem Sumpfprodukt der Destillierkolonne (D1),
eine Destillierkolonne (D2) zum weiteren Entwässern der extrahierten Mischung aus Ameisensäure und Extraktionsmittel,
eine Destillierkolonne (D3) zum Abdestillieren des Extraktionsmittels aus dem Sumpfprodukt der Destillierkolonne (D2),
gekennzeichnet durch
mindestens einen Seitenabzug und einen Kopfabzug an der Destillierkolonne (D3) zum Entnehmen von Ameisensäuren unterschiedlicher Konzentration.

10. Vorrichtung nach Anspruch 9, gekennzeichnet durch die Ausbildung der Destillierkolonnen (D1) und (D2) als eine Destillierkolonne.

## Claims

1. A process for obtaining anhydrous or substantially anhydrous formic acid by hydrolysis of methyl formate, in which
a. methyl formate is subjected to hydrolysis,
b. the methanol and the excess methyl formate are removed from the resultant hydrolysis mixture by distillation,
c. the bottom product from the distillation (b), consisting of formic acid and water, is extracted in a liquid-liquid extraction with an extractant which dissolves principally the formic acid,
d. the resultant extract phase, consisting of formic acid, the extractant and some of the water, is subjected to distillation,
e. the top product obtainable from this distillation, which consists of all or some of the water introduced into the distillation and some of the formic acid, is fed back in vapor form into the lower section of the distillation column in step (b),
f. the bottom product from distillation step (d), which consists of the extractant, possibly some of the water and the majority of the formic acid, is separated by distillation into variously concentrated liquid streams of anhydrous or substantially anhydrous formic acid on the one hand and the extractant on the other hand,
which comprises removing, in step (f), one or more liquid streams having a formic acid content of from 80 to 95% by weight via at least one side outlet and a liquid stream having a formic acid content of at least 95% by weight via a head outlet.

2. A process as claimed in claim 1, wherein the extractant leaving step (f) is fed back into the process.

3. A process as claimed in claim 1, wherein the distillation steps (b) and (d) are carried out in a single column which fulfils the functions of the columns in these steps.

4. A process as claimed in claim 1, wherein the water needed for the hydrolysis is introduced in vapor form into the lower section of the column in step (b).

5. A process as claimed in claim 2, wherein the water needed for the hydrolysis is introduced in vapor form into the central section of the column.

6. A process as claimed in claims 1 to 4, wherein the hydrolysis (a) is carried out using methyl formate and water in a molar ratio of from 1:2 to 1:10.

7. A process as claimed in claims 1 to 5, wherein the extractant used is a carboxamide of the formula I 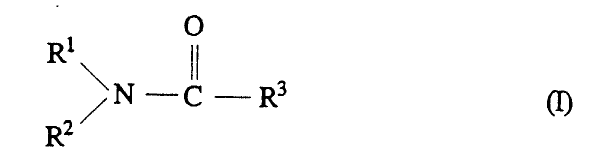 where the radicals R¹ and R² are alkyl, cycloalkyl, aryl or aralkyl groups or together are a 1,4- or 1,5-alkylene group, in each case having 1 to 8 carbon atoms, with the proviso that the total number of carbon atoms in R¹ and R² is from 7 to 14 and that only one of the radicals is an aryl group, and R³ is hydrogen or a C₁-C₄-alkyl group, preferably hydrogen.

8. A process as claimed in claims 1 to 6, wherein, if an extractant (I) is used, the hydrolysis (a) is carried out in the presence of the extractant.

9. An apparatus for carrying out the process as claimed in any of the process claims, which comprises:
a reactor (H) for the hydrolysis of methyl formate,
a distillation column (D1) for the removal of methanol from the hydrolysis mixture by distillation,
an extraction column (E) for the extraction of water from the bottom product from the distillation column (D1),
a distillation column (D2) for further removal of water from the extracted mixture of formic acid and extractant,
a distillation column (D3) for the removal of the extractant from the bottom product from the distillation column (D2) by distillation,
wherein the distillation column (D3) has at least one side outlet and one head outlet for the removal of formic acids in different concentrations.

10. An apparatus as claimed in claim 9, wherein the distillation columns (D1) and (D2) are designed as a single distillation column.

## Revendications

1. Procédé d'obtention d'acide formique dépourvu d'eau ou en grande partie dépourvu d'eau par l'hydrolyse du formiate de méthyle,
conformément auquel
a. on soumet le formiate de méthyle à l'hydrolyse,
b. on élimine par distillation le méthanol, ainsi que le formiate de méthyle excédentaire du mélange d'hydrolyse obtenu,
c. au cours d'une extraction liquide-liquide avec un agent d'extraction absorbant principalement l'acide formique, on soumet à extraction le produit de fond ou queue de la distillation (b) qui se compose d'acide formique et d'eau,
d. on soumet à distillation la phase d'extrait ainsi obtenue, qui se compose d'acide formique, de l'agent d'extraction et d'une partie de l'eau,
e. on ré-introduit sous forme de vapeur dans la partie inférieure de la colonne de distillation de l'étape (b) le produit de tête obtenu au cours de cette distillation de l'étape ci-dessus qui se compose de la quantité totale ou d'une quantité partielle de l'eau introduite dans la distillation et d'une parte de l'acide formique,
f. on sépare par distillation le produit de fond ou queue qui se compose de l'agent d'extraction, éventuellement d'une quantité partielle de l'eau et de la plus grande partie de l'acide formique de l'étape de distillation (d) en courants liquides différemment fortement concentrés de l'acide formique dépourvu d'eau ou en grande partie dépourvu d'eau, ainsi qu'en l'agent d'extraction,
caractérisé en ce que
dans l'étape (f), on prélève par au moins une sortie latérale un ou plusieurs courants liquides avec une fraction d'acide formique de 80 à 95% en poids et par une sortie de tête un courant liquide avec une fraction d'acide formique d'au moins 95% en poids.

2. Procédé suivant la revendication 1, caractérisé en ce que l'on recycle l'agent d'extraction qui quitte l'étape (f) dans le processus opératoire.

3. Procédé suivant la revendication 1, caractérisé en ce que l'on entreprend les étapes de distillation (b) et (d) dans une seule colonne qui remplit les fonctions des colonnes de ces étapes.

4. Procédé suivant la revendication 1, caractérisé en ce que l'on introduit l'eau nécessaire à l'hydrolyse sous forme de vapeur dans la partie inférieure de la colonne de l'étape (b).

5. Procédé suivant la revendication 2, caractérisé en ce que l'on introduit l'eau nécessaire à l'hydrolyse sous forme de vapeur dans la partie médiane de la colonne.

6. Procédé suivant l'une quelconque des revendications 1 à 4, caractérisé en ce que, au cours de l'hydrolyse (a), on utilise le formiate de méthyle et l'eau dans le rapport molaire de 1:2 à 1:10.

7. Procédé suivant les revendications 1 à 5, caractérisé en ce que l'on utilise, à titre d'agent d'extraction, un carboxamide de la formule générale I 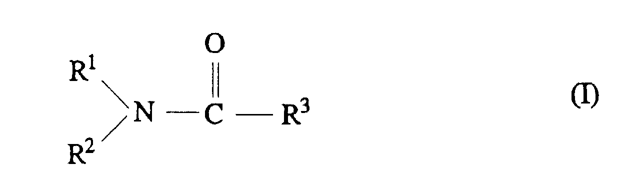 dans laquelle les symboles R¹ et R² représentent chacun un radical alkyle, cycloalkyle, aryle ou aralkyle ou forment en commun, un radical 1,4- ou 1,5-alkylène qui comporte à chaque fois de 1 à 8 atomes de carbone, avec la condition que la somme des atomes de carbone de R¹ et de R² varie de 7 à 14 et que seul un des restes représente un groupe aryle et dans laquelle R³ représente de préférence l'hydrogène ou un groupe alkyle en C₁ à C₄.

8. Procédé suivant les revendications 1 à 6, caractérisé en ce que, dans le cas de l'emploi d'un agent d'extraction (I), on entreprend l'hydrolyse (a) en présence de l'agent d'extraction.

9. Installation pour entreprendre le procédé suivant l'une quelconque des revendications précédentes, où l'installation comporte :
un réacteur (H) pour l'hydrolyse du formiate de méthyle,
une colonne de distillation (D1) pour l'élimination par distillation du méthanol hors du mélange d'hydrolyse,
une colonne d'extraction (E) pour l'extraction d'eau hors du produit de fond ou queue de la colonne de distillation (D1),
une colonne de distillation (D2) pour la déshydratation plus poussée du mélange extrait d'acide formique et d'agent d'extraction,
une colonne de distillation (D3) pour l'élimination par distillation de l'agent d'extraction hors du produit de fond ou queue de la colonne de distillation (D2),
qui se caractérise par
au moins une sortie latérale et une sortie de tête sur la colonne de distillation (D3) pour le prélèvement d'acides formiques de concentrations différentes.

10. Installation suivant la revendication 9, caractérisée par la conformation des colonnes de distillation (D1 ) et (D2), sous la forme d'une colonne de distillation.
